# EUROPEAN PATENT APPLICATION

(11) **EP 3 821 924 A1**
(43) Date of publication of application: **19.05.2021**
(21) Application number: 20207046.2
(22) Date of filing: 11.11.2020
(51) Int. Cl.: A61M 5/20, A61M 5/315, A61M 5/24, A61M 5/28

(54) **PRE-FILLED SYRINGE THAT COMPRISES AN ELASTIC AND SLIDING VALVULAR JOINT**

(30) Priority: 12.11.2019 AR P190103308
(71) Applicant: Szapiro, Jaime Luis, City of Buenos Aires, Argentina C1425FRF (AR); Szapiro, Germán Andrés, City of Buenos Aires, Argentina C1173ACF (AR); Moreno Bonino, Saúl, Province de Buenos Aires, Argentina B1746QQA (AR)
(72) Inventor: Szapiro, Jaime Luis, City of Buenos Aires, Argentina C1425FRF (AR); Szapiro, Germán Andrés, City of Buenos Aires, Argentina C1173ACF (AR); Moreno Bonino, Saúl, Province de Buenos Aires, Argentina B1746QQA (AR)
(74) Representative: Fernández-Pacheco, Aurelio Fernández

(57) **Abstract**

Pre-filled syringes, which comprises a single chamber with an injectable product or two chambers that contain isolated products to be mixed before the injection, and an elastic and sliding valvular joint acting as a temporary closure to prevent the internal chamber from communicating with the outside, or both internal chambers from communicating between them. Said joint comprises two discoid cooperative elements: an elastic discoid cap and a sliding receiving discoid seat, including a central passage tube. Its edge forms a cord that is thicker and less elastic that the rest of the seat. The cap includes an elastic base from which a compact closing cylinder is formed with a distal area with a plurality of longitudinal grooves and a proximal fit area. The elastic base of the cap is equivalent to the base of the receiving discoid seat. The cap can: (a) half-fit, which will allow the communication of fluid through the plurality of grooves, and (b) completely fit the central tube of the receiving discoid seat. Both cooperative discoid elements do not completely separate from each other while being used. In this way, the valvular joint at a half-fit position allows the lyophilization of the content of the syringe with the valvular joint set inside the syringe. The valvular joint is manufactured with an elastomeric material either by injection or compression.

## Description

### FIELD OF THE INVENTION

The present invention makes reference to the field of syringes to apply injections. Preferably, it makes reference to those pre-filled and disposable syringes, and even more particularly it refers to an elastic and sliding valvular joint for pre-filled disposable syringes to maintain the contents therein completely isolated and sterile to prevent them from reaching the needle before the moment of the injection.

### BACKGROUND OF THE INVENTION

Generally, pre-filled syringes include a cylindrical and hollow main body, with a front section where a communication neck is formed with the injection needle; while on the inside there is a plunger coaxially arranged and manually movable. This plunger extends outwards from the base opposite to the neck, which is totally open.

Under these conditions, an internal chamber of variable volume is defined. This chamber constitutes the temporary location for the product to be injected. Said chamber is determined by the cylindrical wall of the main body, the cited front neck, where the needle couples, and the active head of the manually movable plunger that is exactly supported by the internal face of said cylindrical wall of the main body.

The needle is in the interior of a protective sheath, which at the same time locks and couples to it to keep it isolated and steady, while it also couples to the syringe itself creating a temporary hermetic closure.

Taking into consideration the basic structure previously described, there are several embodiments where the mentioned interior of the main body is also subdivided in two chambers of variable volume that are adjacent and coaxial, and separated between them by internal transversal partitions, which include valvular means to enable the communication between both chambers.

It makes reference to the usually called pre-filled double-chamber syringes, which are used for those cases where two different liquid products are stored, or when one liquid product and another powdered product must be kept completely isolated and separated one from the other until the moment of injection, when they are previously mixed.

Indeed, in the case of double-chamber syringes said internal valvular means, which constitute the partition that separates both chambers, are designed for the user to mix the products before the injection without being contaminated by the outer environment. That is to say, it maintains the isolation from origin. It is then that the protective sheath can be removed from the needle and, the resulting mix can be moved to the needle, initiating the injection process.

In order to carry out said mixing step, while maintaining the mentioned isolation, it is usual and common to use the plunger of the syringe by moving it in a direction opposite to the direction of the injection. This moving action generates pressures and depressions in said adjacent chambers; and therefore, the mentioned internal valvular means open, establishing a communication that allows one of the products to pass and mix with the other, and then placing the mix in only one chamber and in the conditions to be injected.

Once the mix is produced, the user will only have to proceed with the injection for the mix to move forward towards the needle.

There are several constructive embodiments that define said internal valvular partitions, which separate the adjacent chambers that contain the separated products. Precisely, said partitions differ by the valvular resource they include in each case.

However, from the analysis of these well-known internal valves, there arises the problem of the valvular means especially designed for each particular case. That is to say, they can only act in the body of a syringe that is structurally and functionally adapted to them.

In other cases, there are valvular elements that can be adapted to the body of conventional syringes, but which require the addition of an accessory or a structural modification to the body of the syringe, which raises their final price and complicates their manufacture and filling.

It is further clarified that in order for these valvular joints to act effectively in pre-filled double-chamber syringes, it is important that the mentioned neck of the syringe, where the injection needle couples with its protective sheath, remains closed by a valve-cap or, otherwise by an adequate element that keeps the liquid isolated in the interior of the needle until the injection. In this manner, it guarantees that the coupling and uncoupling actions will not cause an unwanted loss or spill.

Within this type of valvular joints, it is possible to find the following Argentine Patent of Invention: AR 082099 A1 (= CN 102861369 A). Its owners are Jaime Luis Szapiro, Saul Moreno and Leonardo Szames and, it is entitled "ELASTIC AND SLIDING VALVULAR JOINT, SUITABLE TO WORK IN PRE-FILLED SYRINGES" ("CONJUNTO VALVULAR ELÁSTICO Y DESLIZANTE APTO PARA ACTUAR EN EL INTERIOR DE JERINGAS PRELLENADAS"). This invention refers to an elastic and sliding valvular joint suitable to work in two types of pre-filled syringes: the ones that include one internal chamber that stores the product to be injected or the ones that include two internal and independent chambers, which hold the corresponding isolated products that must be mixed before the injection. This valvular joint acts as a temporary closure that prevents said internal chambers from communicating between them or with the communication tube towards the injection needle. The joint consists of two discoid cooperative elements arranged inside the main body of the syringe, where their perimeter edges are supported by the cylindrical surface of the body. One of said elements is an elastic and sliding discoid cap, while the other constitutes the receiving sliding discoid seat on which said cap acts. The perimeter edges of the bases of both discoid elements determine each cord, which is thicker and less elastic that the rest of the body of each valvular element.

The elastic and sliding discoid cap comprises an elastic base with a plurality of holes, and a hollow closing cylinder with an open distal base is formed from the internal face of said elastic base; while, the sliding receiving discoid seat includes a central passage tube, which is opposite to said closing cylinder, having its inner entrance enclosed between the outer cavities that maintain the communication with the interior of the tube.

Additionally, the closing cylinder that is formed from the elastic base of the valve cap has an external diameter of its round section slightly bigger than the diameter of the passage tube of the receiving discoid seat.

Due to the design of this valvular joint, its elements can only be produced by thermoplastic injection. Therefore, the only construction material to be used will be an elastomeric product that allows the manufacturing by injection, such as santoprene. Once the elements of this valvular joint are injected, their lifetime is relatively short since they tend to lose their elastic memory through time. This phenomenon prevents said elements from the valvular joint to separate correctly, or once separated they tend to easily reassemble. This fact has negatively affected the functioning of the invention.

Patent application AR 100575 A3 refers to a valvular resource that comprises two cooperative discoid elements arranged inside the main body of the syringe with their perimeter edges supported by the cylindrical surface of said body. One of said elements is an elastic sliding discoid cap, while the other is a sliding receiving discoid seat, where the cap acts. The perimeter edges of the bases of both discoid elements determine cords that are thicker and less elastic than the rest of the body of each valvular element. The elastic sliding discoid cap comprises a closed plain base, and from its internal face a hollow closing cylinder with an open distal base is formed. Its thicker perimeter cord is affected by the lateral peripheral slots that constitute the corresponding passage tubes of the product during its functioning. On the thicker perimeter cord two or more lateral peripheral slots are determined equidistant from each other. And, they have the same height as the thickness of the cord.

In this application a valvular resource is described with the intent to obtain an independent valvular joint made of a unique material such as santoprenre, and an exclusive manufacturing method. Also, it intends to eliminate the holes in the plane body of the disc that comprises the cap and change it for two perimeter slots. This resource weakens the perimeter edge of the disc, which must be thicker and less elastic than the rest of the body, and therefore, it affects its functioning. Additionally, the passage of the product through the mentioned slots is not substantially improved. In particular, we looked for versatility in the materials and in the manufacturing method as well as an improvement in the volume of flow through the perimeter slots, but the perimeter cord that fits said disc against the wall of the syringe weakened.

Furthermore, we can mention the Argentine Patent Application AR 026723 B1 (= EP 1213036 B1, ES 2254321 T3), owned by Jaime Luis Szapiro, Saul Moreno and Leonardo Szames. It is entitled "Elastic and Sliding Valvular Joint suitable to work in the interior of Pre-filled Syringes" ("Conjunto Valvular Elástico y Deslizante Apto para Actuar en el Interior de Jeringas Prellenadas"). It referred to syringes that include one internal chamber that stores the product to be injected or two internal and independent chambers that hold the corresponding isolated products that must be mixed before the injection. The valvular joint acts as a temporary closure that prevents said internal chambers from communicating with each other or with the communication tube towards the injection needle.

The joint comprises two cooperative discoid elements that are arranged inside the main body of the syringe, and their perimeter edges supported by the cylindrical surface of the body. One of said elements is an elastic and sliding discoid valve, while the other is a sliding discoid seat on which the discoid valve acts. This discoid valve has an elastic base with a plurality of holes from where a stub is formed. This stub is opposite to a corresponding passage hole that is defined in the base of said sliding discoid seat.

The perimeter edges of the bases of both discoid cooperative elements determine thicker and less elastic cords than the rest of the body of each valvular element.

According to this disclosure, the mentioned discoid cooperative elements, which are arranged on the internal communication between the main body and the neck of the syringe, may act as a resource of temporary closure for pre-filled single-chamber syringes; or if they are combined with a valvular cap that closes the neck of the syringe, they may act as a resource of temporary closure for pre-filled syringes with two internal coaxial chambers acting as a separating partition between them.

It has been verified that in this type of disposable syringe, after mixing the product to be injected and moving the plunger of the syringe to perform the injection, the mentioned elastic and sliding discoid valve, which holds the closing stub of the passage that defines the sliding discoid seat, releases said passage. However, it tends to return to its closing position.

In effect, in some cases, when the user proceeds with the injection, he/she detects that the mentioned closing stub wrongfully enters the passage hole of the discoid seat, acting as an unexpected cap and making it necessary to repeat the initial handling by moving the plunger backwards again and, hence releasing the passage again.

This occurs due to the mentioned closing stub that keeps aligned with the referred passage hole. Although it has a diameter slightly bigger than said hole, it generates an undesired blockage when it settles due to the pressure produced by the plunger.

In this case it would be enough to restart the filling action by moving the plunger of the syringe in a direction opposite to the injection in order for the stub to uncouple.
Furthermore, due to the design of the above mentioned valvular joint the elements can only be manufactured by thermoplastic injection. This implies that the only construction material to be used will be an elastomeric product that allows the manufacturing by injection, such as santoprene. Once this type of products are injected their lifetime is relatively short since they tend to lose their elastic memory through time. This phenomenon prevents the elements of the invention from separating correctly, or once separated they tend to easily reassemble. This fact negatively affects the functioning of the invention.

Also, Patent Application EP0974373A1 (= ES2211014T3) makes reference to a syringe with two chambers of variable volume inside the main cylindrical body. Said chambers are separated by a displacing valve joint, and they can store components that must be mixed before the injection The novelty resides in the special structure of the parts and elements that constitute said valve joint, which comprises two displacing and cooperative elastomer portions. One of said parts is a peripheral and hollow cylindrical body supported by the cylindrical wall of the main body. The other part is a solid and internal cylindrical body that displaces through the internal face of the front part.

The internal part of the valvular joint mentioned in this document is solid, and its structure makes it possible to fit inside the body of the first part of the valve. Said part is supported by the internal cylindrical wall of the syringe. Its structure and disposition intends to avoid displacements while keeping the joint aligned during the bolt considering that it is a solid body with poor flexibility, and it must allow the free flow of liquid during the mixing of the components and then its exit towards the needle. The complexity of both elements of the valvular joint described herein prevents the versatility of the materials and of the processes that facilitates their correct manufacturing and ideal functioning.

Finally, Patent Application US5643224A (= ES2087835B1) refers to a cap to make pre-filled syringes. It is a distintinctive and completely different device that has been created for such end. In conclusion, it has nothing to do with the main or secondary object of this invention.

Indeed, this document refers to a safety valve-cap, which can be applied to disposable pre-filled syringes. It consists of a solid body, substantially cylindrical that has a conical end and a head, where its cylindrical area has a transverse section bigger than the rest of the body. As a continuation of the conical area there are two faceted parts that extend towards the close area of the head. Said faceted parts are equal, parallel and symmetric as well as coaxial to the longitudinal axis of the cap. The cylindrical head has two faceted parts that are equal, parallel and symmetric. These parts are parallel to the faceted parts of the cylindrical body. Therefore, this patent refers to a cap that exclusively functions in the outlet hole of any type of syringe without making a valvular joint to be used inside pre-filled syringes.

There was an intent to look for a valvular joint with a simplified design that would allow a more effective functioning. Also instead of obtaining the elements of the valvular joint from the manufacturing by injection of an elastomer, such as the santoprene, which is a thermoplastic rubber that is processed in machines for plastics but with the final appearance of a rubber, there was an intent to use other manufacturing methods that are being currently used in Pharmacotechnology, such as by the compression of elastomer and other materials for medical use that are more economic like butyl rubber, which is a synthetic copolymer Isobutylene Isoprene Rubber (IIR). In the same way, when a solution to be injected (for example, a powder with a diluted active in an adequate solvent) results from the mixing process and moves to the upper chamber of the pre-filled syringe, or when the solution to be injected of a pre-filled syringe is in one single chamber, we looked for a valvular joint that would allow the free flow of the solution to be injected through a wide tube and not through small holes; so that consequently and with the force of the plunger, the components of the valvular joint could act together and assemble with the plunger in the first case, or they could assemble again when finalizing the injection in the second case.

Patent Application EP3473284 (A1) was filed with the intent to accomplish these objectives. It referred to a pre-filled syringe with one internal chamber that stores the product to be injected or two internal and independent chambers that hold the corresponding isolated products that must be mixed before the injection. The syringe includes an elastic and sliding valvular joint suitable to act inside said pre-filled syringes. Said valvular joint acts as a temporary closure that prevents the communication between the internal chamber and the outside or between said internal chambers. The joint comprises two cooperative discoid elements that are arranged inside the main body of the syringe against the internal surface. One of said elements is an elastic discoid cap the other is a receiving and sliding discoid seat. The cap acts in a cylindrical cavity. The edge of the discoid seat forms a cord that is thicker and less elastic than the rest of the seat. The cap comprises an elastic base from which an open hollow and closing cylinder is formed. The receiving discoid seat includes a central passage tube facing the hollow cylinder. The elastic base of the cap fits in a removable manner inside one of the cylindrical cavities of the opening of the central passage tube of the seat. The diameter of the elastic base of the cap is equivalent to the diameter of the receiving discoid seat. When the hollow closing cylinder is inside the central passage tube of the discoid seat, the elastic base of the cap fits inside a circular cavity surrounded by the edge of the discoid seat that forms said cord. The outer diameter of the hollow closing cylinder is equivalent to the internal diameter of the central passage tube of the receiving discoid seat, offering a calibrated fit.

However, it is still desirable to have a pre-filled syringe with an internal valvular joint easily added, which means that a half-assembled valvular joint could be added easily. This would allow the flow of inner air without pressure and/or without the help of special mechanical devices. Also, given the condition of the half-assembled valvular joint and considering it is inside the body of the syringe, it would allow the lyophilization of the content of the proximal chamber whenever the products requires it. This means that it would be ideal if the syringe has the versatility to transform into a container of the pharmaceutical product to be injected and that is likely to be lyophilized inside the syringe itself during the filling process and with a valvular joint already installed inside the body of the syringe. It is also important to have a pre-filled syringe with a valvular joint that does not completely separate during its use so that its content can flow from one chamber to another when applying pressure or suction with the plunger, or when applying the liquid to be injected to a patient. This is not possible with the pre-filled syringes from the state of the art. It is a technological advance of extreme importance since it completely modifies the preparation and filling of this type of syringes. Also, it would be ideal if said valvular joint could be manufactured with any elastomer, whether by compression or injection process.

### SUMMARY OF THE INVENTION

Therefore, the main object of this invention is a pre-filled syringe with either an internal chamber that stores the product to be injected, or two internal and independent chambers that contain isolated products that must be mixed before the moment of the injection, and which comprises an elastic and sliding valvular joint suitable to act in the interior of said pre-filled syringe. This valvular joint acts as a temporary closure that prevents an internal chamber from communicating with the communication tube towards the injection needle or both internal chambers from communicating with each other. Said valvular joint consists of two cooperative discoid elements arranged inside the main body of the syringe with its perimeter edge supported by its cylindrical surface. One of these elements is an elastic discoid cap, while the other is a receiving and sliding discoid seat on which said cap acts through a cylindrical central passage tube. The perimeter edge of the discoid seat forms a cord that is thicker and less elastic than the rest of the body of said discoid seat. The elastic discoid cap of said valvular joint comprises an elastic base, and a compact closing cylinder is formed from the internal face of said elastic discoid base. The compact closing cylinder has a distal area that comprises a plurality of longitudinal grooves and a proximal fit area. The sliding receiving discoid seat includes a central passage tube, which is opposite to said compact closing cylinder in such a way that the elastic base of the cap fits in a removable manner inside an internal perimeter slot located in the opening of the central passage tube of the sliding receiving discoid seat. The diameter of the elastic base of the elastic discoid cap is smaller than the diameter of the receiving and sliding discoid seat. When the compact closing cylinder is inside the central passage tube of the receiving and sliding discoid seat, the elastic base of the elastic discoid cap fits inside a circular cavity surrounded by the edge of the discoid seat that forms a cord. The cord is thicker so it can adopt two positions: (a) half-fit, where the external diameter of the proximal area of fit of the compact closing cylinder is exposed and the distal area that is equivalent to the internal diameter of the central passage tube of the receiving discoid seat is half-fitted. This allows the communication of fluid through the plurality of grooves on both sides of the valvular joint, and (b) completely fit when the compact closing cylinder completely fits inside the central passage tube of the receiving discoid seat preventing the fluids on both sides of the valvular joint from communicating, and where the external diameter of the proximal fit area of the compact closing cylinder is equivalent to the internal diameter of the central passage tube of the receiving discoid seat providing an adjusted fit. The cooperative discoid elements of the valvular joint are manufactured with an elastomeric material either by injection or compression.

In a preferred form, said communication of fluid through the plurality of grooves on both sides of the valvular joint is in a (a) half-fitted position, which allows the lyophilization of the content of the syringe with the valvular joint already installed inside the body of the syringe.

Also in a preferred form, the compact closing cylinder formed from the elastic base of the elastic discoid cap has a circular section, where the quantity of grooves of the plurality of grooves from the distal zone of the compact closing cylinder is from two to six, preferably four.

As an alternative form, the compact closing cylinder formed from the elastic base of the elastic discoid cap has a circular section, where the external diameter in the proximal area of fit of the compact closing cylinder is slightly bigger than the diameter of the central passage tube of the receiving and sliding discoid seat providing an adjusted fit.
Additionally as an alternative form, the compact closing cylinder formed from the elastic base of the valvular cap includes in the proximal fit area a plurality of external annular flanges that are temporary supported by the cylindrical surface that defines the central passage of the sliding receiving discoid seat providing an adjusted fit.

Preferably, the cooperative discoid elements are combined with a tip-cap seal that closes the communication tube towards the injection needle, which is not pre-installed in its position. This is a temporary closing resource for pre-filled syringes of double internal coaxial chambers, where said discoid cooperative elements are arranged in a way that they act as a separating partition between both chambers.

In a preferred embodiment, the cooperative discoid elements are arranged as a temporary closure that prevents the main body of the syringe from internally communicating with the communication tube towards the injection needle, which is a temporary closure resource for pre-filled syringes with a single chamber.

In another embodiment, the elastic valvular joint delimits two chambers that each one comprises a different product, and at least one of them is liquid, and when mixing both products they create a solution to be injected, where the elastic base of the elastic discoid cap of the elastic valvular joint is placed opposite to the head of the plunger.

Particularly, the elastic valvular joint delimits a chamber that comprises a solution to be injected, where the elastic base of the elastic discoid cap of the elastic valvular joint faces the head of the plunger.
More particularly, the injection needle is pre-installed in the exit tube of the syringe.

### BRIEF DESCRIPTION OF THE DRAWINGS

In order to precise the advantages perfunctorily mentioned and that the users or specialists could possibly add, and for better comprehension of the constructive, functional and structural characteristics of this invented elastic valvular joint, a preferred embodiment is described below and illustrated in a schematic manner and without a specific scale, in the sheets of drawings attached herewith. It is important to state clear that since it is an example, its scope of protection should not be considered limiting or exclusive. Instead, it is simply aimed to explain and illustrate the basic understanding upon which the invention is based.
- Figure 1 is a cross-section view that shows a preferred embodiment of the elements that form the elastic valvular joint of a pre-filled syringe of this invention in a half-fitted position.
- Figure 2 is a cross section view of the valvular joint of previous Figure 1, but in this case it shows the same fitted elements coupled between them.
- Figure 3 is a perspective view that represents the elastic discoid valvular cap that constitutes one of the two elements of the valvular joint of the pre-filled syringe of this invention as seen from its external face.
- Figure 4 is a perspective view that represents the elastic discoid valvular cap of previous Figure 3 that constitutes the other element of the valvular joint of this invention as seen from its internal face.
- Figure 5 is a perspective view that represents the sliding receiving discoid seat that constitutes the other element of the valvular joint of the invention as seen from its external face.
- Figure 6 is a perspective view that represents the sliding receiving discoid seat of previous Figure 5 as seen from its internal face.
- Figure 7 is a vertical lengthwise cross-section view that represents a preferred embodiment of a pre-filled double-chamber syringe of variable volume, in which interior the valvular joint is arranged at a rest position as it is offered in the commerce.
- Figure 8 is also a lengthwise cross-section view of the pre-filled syringe of previous Figure 7, which shows the valvular joint in the position after the mixing process is initiated inside the syringe of the invention.
- Figure 9 is also a lengthwise cross-section view of the pre-filled syringe of previous Figure 8, which shows the valvular joint in the position after the mixing process is finished inside the pre-filled syringe of this invention.
- Figure 10 is a lengthwise cross-section view of the pre-filled syringe of previous Figure 9, which shows said syringe with the needle on top and in the conditions to start the process of injecting the products of both mixed chambers.
- Figure 11 is a lengthwise cross-section view of the pre-filled syringe of previous Figure 10, which shows said syringe with the needle on top during the process of injecting the products of both mixed chambers.
- Figure 12 is a lengthwise cross-section view of the pre-filled syringe of previous Figure 11, which shows said syringe with the needle on top finalizing the process of injecting the products of both mixed chambers.
- Figure 13 is a lengthwise cross-section view that represents a preferred embodiment of the pre-filled syringe with a single chamber of variable volume of this invention, which includes a needle on top covered by its protective sheath, and in its interior there is a valvular joint in rest position as it is offered in the commerce, but in this case said valvular joint acts as a seal that prevents the content of the pre-filled syringe from entering the tube of the needle.
- Figure 14 is a vertical lengthwise cross-section view that represents a pre-filled syringe of previous Figure 13, where the needle on top is uncovered, showing the behavior of valvular elements when the F force is applied with the plunger outwards the syringe, moving the cap of the valvular joint before the injection process.
- Figure 15 is a vertical lengthwise cross-section view that represents a pre-filled syringe of previous Figure 14, which shows the behavior of the valvular elements when the product of the pre-filled syringe is moved through the injection and the needle during the injection process.
- It is further clarified that in all the figures the same reference numbers correspond to the same or equivalent parts or elements forming the joint, according to the embodiment selected for this explanation of the invented valvular joint.

### DETAILED DESCRIPTION OF THE INVENTION

The pre-filled syringes of this invention comprise a valvular joint that belongs to the group of those syringes that adapt to all types of body of the conventional syringe, whether single-chamber or double-chamber.

The constructive and functional design of this valvular joint takes into account the condition of adapting to all types of conventional syringes as well as the non-alteration of automatized processes for getting the products inside the syringe, especially for the cases of double-chamber pre-filled syringes, and also the lack of special and/or complicated tasks demanded to the user when mixing and then injecting.

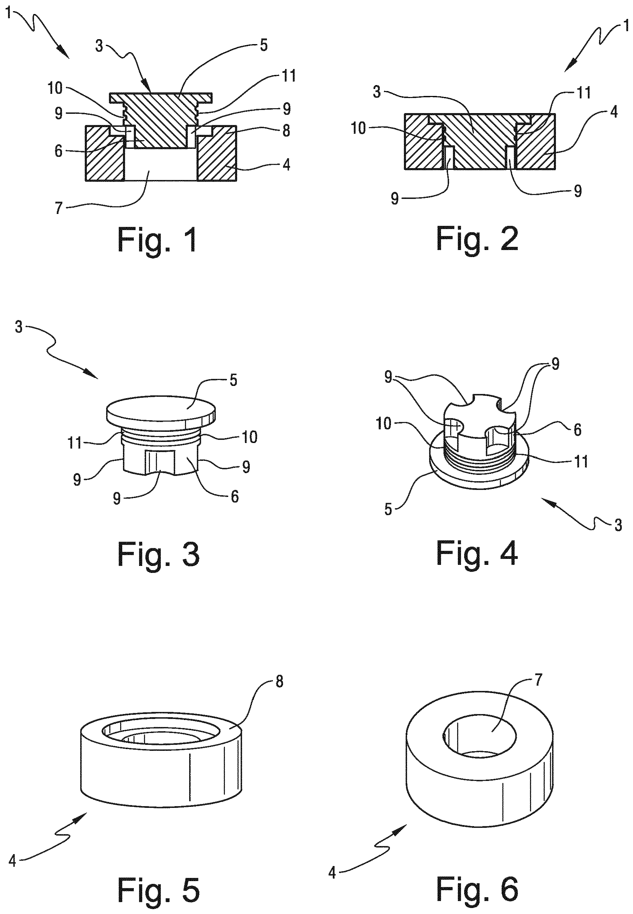

As it is observed from Figures 1 to 6, the elastic and sliding valvular joint (1) for syringes (2) previously filled referred to in this invention consists of two mutually cooperative elements represented by (3 and 4).

The one indicated with reference (3) is an elastic discoid cap that consists of an elastic discoid base (5) substantially formed in a cylindrical manner.
This elastic discoid cap (3) stands out especially because a compact closing cylinder (6), which is coaxial with the mentioned elastic discoid base (5), is formed from the internal face of the elastic discoid base (5). The compact closing cylinder (6) has a distal area that comprises a plurality of longitudinal grooves (9) and a proximal fit area (10).

Another element of the valvular joint (1) is a receiving discoid seat (4) that includes a central passage tube (7) facing said compact closing cylinder (6) in a way that the elastic base (5) of the elastic discoid cap (3) fits in a removable manner within the inner perimeter slot located in the opening of the central passage tube (7) of the sliding receiving discoid seat (4).

Said sliding receiving discoid seat (4) was created in order for the elastic discoid cap (3) to produce the closure or opening of the valvular joint (1), acting as a cooperative receiving discoid seat. This element is also preferably circular, and it has a central passage tube (7) and a corresponding perimeter ring (8) thicker than the rest of the body of said element.

The elastic discoid cap (3) and the sliding receiving discoid seat (4) of the valvular joint (1) are cooperative elements because they necessarily act together to perform the valvular closure and opening.

To this extent, said elements (3 and 4) must be overlapped and aligned between them inside the syringe; so that the mentioned closing cylinder (6) perfectly fits the interior of the central passage tube (7) generating the hermetic closure that prevents the products contained inside the syringe (2) previously filled from moving through said tube. Said proper fit inside the central passage tube (7) is obtained through a bigger diameter of the closing compact cylinder (6).

The elastic base (5) of the elastic discoid cap (3) has a smaller diameter than the one from the elastic discoid cap (3), and when the compact closing cylinder (6) is inside the central passage tube (7) of the receiving discoid seat (4), said elastic base (5) of the elastic discoid cap (3) is fitted inside a circular cavity surrounded by the perimeter ring (8) of the receiving discoid seat (4) that forms a thicker cord.

In this manner, the elastic discoid cap (3) of the valvular joint (1) can adopt two positions: (a) half-fit, where the external diameter in the proximal area of fit (10) of the compact closing cylinder (6) is exposed and the distal area equivalent to the internal diameter of the central passage tube (7) of the receiving discoid seat (4) is half-fitted, which will allow the communication of fluid through the plurality of grooves (9) on both sides of the valvular joint (1), and completely fit when the compact closing cylinder (6) completely fits inside the central passage tube (7) of the receiving discoid seat (4) preventing the fluids on both sides of the valvular joint (1) from communicating, and where the external diameter of the proximal fit area (10) of the compact closing cylinder (6) is equivalent to the internal diameter of the central passage tube (7) of the receiving discoid seat (4) providing an adjusted fit.

An alternative embodiment will be that said bigger diameter of the compact closing cylinder (6) is obtained by a plurality of external annular flanges (11) that are temporary supported by the cylindrical surface that defines the central passage tube (7) of the sliding receiving discoid seat (4).

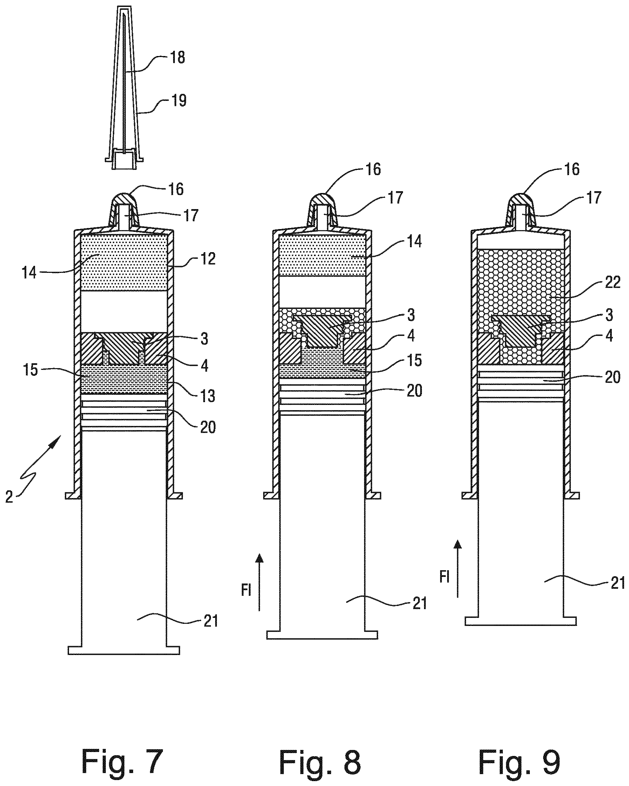

As from Figures 7 to 12, it is possible to understand how the valvular joint of this invention acts in a preferable embodiment, when it is applied to a pre-filled syringe (2) with two chambers (12 and 13) during the process of mixing products (14 and 15) contained in both chambers (12 and 13).

Indeed, in Figure 7 it is possible to observe, in a lengthwise cross-section view, a syringe (2), which is conventional and pre-filled as it is before the injection conditions, i.e. with the corresponding products (14 and 15) duly separated and isolated between them and from the outside, and stored in the corresponding chambers (12 and 13) of variable volume inside the body of the syringe (2).

By means of an upper closing cap (16), also known as "tip-cap", which is in the exit tube (17) of the syringe (2), it is possible to guarantee the airtightness and hence, the duly isolation of the commercial product to be injected.

As it is already known, the syringe (2) comprises its corresponding needle (18) of injection, which is covered by a protective sheath (19) that usually accompanies the commercial product in its container.

In said Figure 7 it can be clearly identified that the upper chamber (12) of the syringe (2), which is closer to the exit of the product during the injection, is delimited in its base by the valvular joint (1) of the invention, in its sides by the internal face of the cylindrical wall of the main body of the syringe (2) and in the upper part by the mentioned closing cap (16), containing the first product (14) duly isolated.

Furthermore, the lower chamber (13) is delimited in its upper part by the same valvular joint (1), by the internal face of the cylindrical wall of the main body of the syringe (2) and by the head (20) of the plunger (21), containing the second product (15) duly isolated.

In the conditions mentioned above, as it is shown in Figure 7, it is possible to store in said chambers (12 and 13) two products, the first one (14) and the second one (15), which may be liquid or solid in powder-form. Preferably, for example one solid in powder-form in the chamber (12) and one liquid in the chamber (13), or both liquids. In both cases they are separately contained in any of the two chambers (12 and 13). The powder can be obtained by lyophilizing a solution that contains said powder inside the syringe (2) itself and through a valvular joint (1) in a half-fitted position in a way that the exit flow of the solvent is determined by the grooves (9) of the elastic discoid cap (3). Preferably, the solid or the liquid locked in the chamber (12) is limited by the valvular joint (1), which is located within the syringe of said half-fitted cap (3) allowing its easy placement by leaving a space between the solid or liquid and the valvular joint (1), until applying vacuum and maintaining the receiving discoid seat (4) fixed until the cap (3) is fitted in the central passage tube (7) that contains said discoid seat (4). When applying pressure with the plunger to the interior of the pre-filled syringe (2), as described later on this space will allow the cap (3) to disengage, and to communicate with both chambers (12 and 13) through the grooves (9) by mixing the products to be injected.

Once the joint is initially formed, while observing said Figures 8 and 9 it is possible to notice the performance of the valvular joint (1) according to a preferred embodiment of this invention, when the internal mixing action of the products (14 and 15) begins before the injection.

For this purpose the user moves the head (20) of the plunger (21) in the direction (FI) of the injection generating pressure on the proximate lower chamber (13), which consequently produces the movement of the elastic discoid cap (3) fitted in the receiving discoid seat (4), being partially disengaged, and hence liberating the communication between both upper and lower chambers (12 and 13). This is possible because when said movement occurs, the product (14) flows through the grooves (9) of the elastic discoid cap (3).

In order to facilitate the obtaining of the solution (22) of the injection, it is preferable that the user vigorously shakes the syringe (2). Said solution (22) must always be duly isolated from the outside through the closing cap (16).

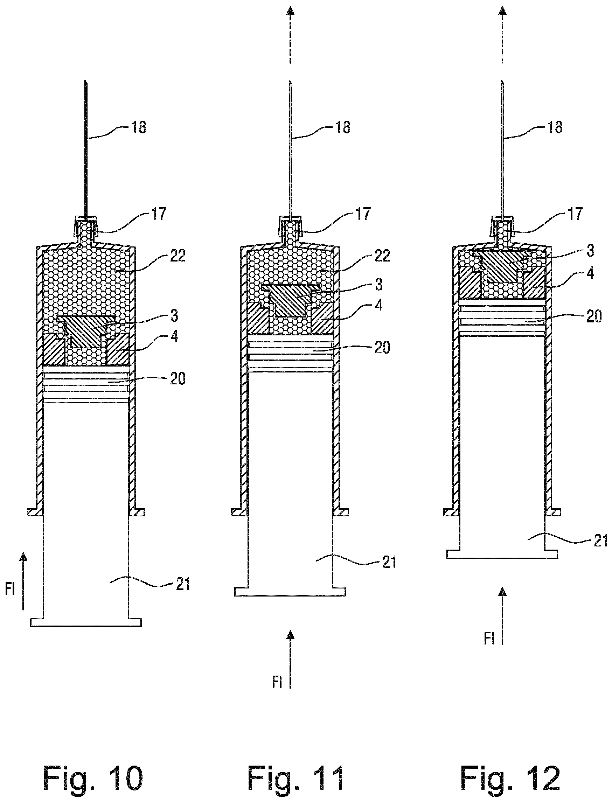

Figure 10 shows the arrangement of the referred elements (3 and 4) and the obtaining of the solution (22) to be injected by mixing the referred products (14 and 15), which is completely located in the upper distal chamber (12) with a needle (18) positioned in the tube (17) of the syringe (2).

It is emphasized the fact that the elastic discoid plug (3) is kept always half-fitted in the central passage tube (7) of the receiving discoid seat (4), and the communication between both internal chambers (12 and 13) is kept through the grooves (9) during the actions to obtain the solution (22) to be injected.

Figures 10, 11 and 12 show that as a consequence of the communication set through the grooves of the distal area of the half-fitted cap (3) in the central passage tube (7), when the user moves the head (20) of the plunger (21) in the direction (FI) of the injection, the mentioned solution (22) moves from the lower or proximate chamber (13) towards the upper or distal chamber (12) in a way that the solution is possible to be injected (22). Said solution was obtained in the same way as any other pre-filled syringe (2) with a single conventional chamber. The pressure (FI) made from the plunger (21) makes the compact closing cylinder (6) of the elastic discoid cap (3) to change to a half-fitted position in the central passage tube (7), which is why the valvular joint (1) works together during the injection of the content of the syringe (2), and in which case it does not affect the flow of fluid. This occurs because the fluid flows perfectly and completely through the grooves (9) because said elastic discoid cap (3) is half-fitted in the tube (7).

Figures 11 and 12 show that once the upper closing cap (16) is withdrawn and the needle (18) of the injection is set in its position in the distal end of the exit tube (17) of the syringe (2) as shown in Figure 10, the user will only have to continue moving the plunger (21) in the direction (FI) such that the pressure applied to the solution itself (22) makes it flow through the needle (19) of the injection by crossing it, after having previously removed the protective sheath (19) from said needle (18).

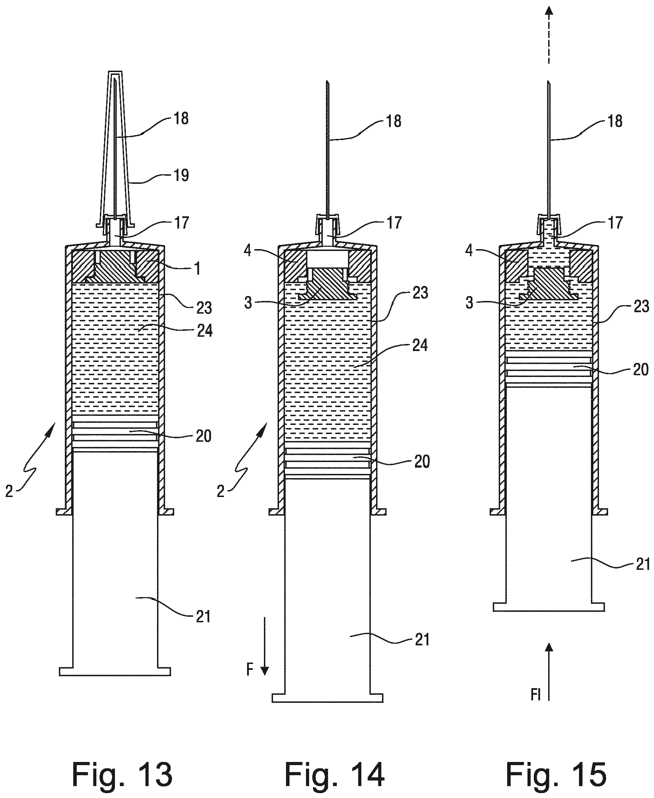

Figures 13, 14 and 15 show the valvular joint (1) according to this invention included in a pre-filled conventional syringe (2) with a single chamber (23).

Indeed, for these cases the valvular joint (1) formed by elements (3 and 4) is placed in the elastic discoid cap (3) facing the plunger (21) of the syringe (2) and closing the internal communication of the syringe (2) with the exit tube (17) of the syringe (2), in such a manner that only the chamber (23) is delimited by the valvular joint (1) as an upper base, and by the cylindrical wall of the main body of the syringe (2) and the head (20) of the plunger (21) as a lower base according to Figure 13.

In this case, the user also moves the plunger (21) in the opposite direction (F) of the injection producing an internal depression that generates the elastic deformation of the elastic discoid cap (3), and hence the movement of the compact closing cylinder (6), which opens a communication through its grooves (9) when changing to a half-fitted position in the central passage tube (7), according to Figure 14. And, when applying an opposite force (FI), the injectable stored solution (24) can exit towards the needle (18) of the injection, and cross it after having previously removed the protective sheath (19) from said needle (18), according to Figure 15.

In both cases mentioned above, whether the pre-filled syringe (2) with two chambers (12 and 13) or with a single chamber (23), the internal communication of the syringe (2) through the exit tube (17) is initially and necessarily blocked by an upper tip-cap (16), which is located in the exit tube (17) of the syringe (2), while the needle (18) of the injection is inside the protective sheath (19) and separated from the syringe (2) inside its corresponding packaging.

In the market there are pre-filled syringes (2), which are commercialized with the needle (18) being previously installed in their exit tube (17). Said syringes (2) may have two chambers (12 and 13) or one single chamber (23) to contain two separate products that are mixed to obtain either one solution (22) to be injected, or one injectable solution (24), respectively. In the case of double-chamber syringes, the exit of the liquid to be injected towards the previously installed needle is through the valvular closing cap as it is described in the invention from Argentine patent AR 250777 V1, owned by Jaime Luis Szapiro, Leonardo Szames and Saul Moreno, and entitled "VALVULAR SAFETY CAP APPLICABLE TO DISPOSABLE PRE-FILLED SYRINGES" ("TAPON VÁLVULA DE SEGURIDAD APLICABLE A JERINGAS PRELLENADAS DESCARTABLES").

In alternative embodiments, the valvular joint (1) can be used in a pre-filled syringe (2) of the invention in a form different to what has been described herein. That is to say, the cap (3) of the valvular joint (1) is facing the head (20) of the plunger (1) of the syringe (2) making the necessary adjustments for the correct functioning.

## Claims

1. Pre-filled syringe (2) with a single internal chamber (23) that stores the product to be injected, or with two independent internal chambers (12 and 13) that contain respectively isolated products (14 y 15), which have to be mixed before the injection, that comprise an elastic and sliding valvular joint (1) suitable to act in the interior of said pre-filled syringe (2), where the valvular joint (1) acts as a temporary closure blocking the communication between the internal chamber (23) and the communication tube (17) towards the injection needle (18), or between the two internal chambers (12 and 13). Said joint (1) comprises two discoid cooperative elements (3 and 4) arranged inside the main body of the syringe (2) and with its perimeter edge supported by its cylindrical surface. While one of said elements is an elastic discoid cap (3), the other forms a sliding discoid receiving seat (4) on which said cap (3) acts through a central passage tube (7), where the perimeter edges of the sliding discoid receiving seat (4) forms a cord, which is thicker and less elastic that the rest of the body of said discoid seat (4). Said pre-filled syringe (2) is characterized because the elastic discoid cap (3) of said valvular joint (1) comprises an elastic base (5) and from its inner face a compact closing cylinder (6) is formed with a distal area that comprises a plurality of longitudinal grooves (9) and a proximal fit area (10), while the sliding discoid receiving seat (4) includes a central passage tube (7) facing said compact closing cylinder (6) in such a way that the elastic base (5) of the discoid cap (3) fits in a removable manner within the inner perimeter slot located in the opening of the central passage tube (7) of the sliding receiving discoid seat (4), where the elastic base (5) of the elastic discoid cap (3) has a smaller diameter than the one from the sliding receiving discoid seat (4). And when the compact closing cylinder (6) is inside the central passage tube (7) of the sliding receiving discoid seat (4), said elastic base (5) of the elastic discoid cap (3) is fitted inside a circular cavity surrounded by the perimeter edge (8) of the discoid seat (4) that forms the ticker cord, and which can adopt two positions: (a) half-fit, where the external diameter in the proximal area of fit (10) of the compact closing cylinder (6) is exposed and the distal area equivalent to the internal diameter of the central passage tube (7) of the receiving discoid seat (4) is half-fitted. This allows the communication of fluid through the plurality of grooves (9) on both sides of the valvular joint (1), and (b) completely fit when the compact closing cylinder (6) completely fits inside the central passage tube (7) of the receiving discoid seat (4) preventing the fluids on both sides of the valvular joint (1) from communicating, and where the external diameter of the proximal fit area (10) of the compact closing cylinder (6) is equivalent to the internal diameter of the central passage tube (7) of the receiving discoid seat (4) providing an adjusted fit. The cooperative discoid elements (3 and 4) of the valvular joint (1) are manufactured with an elastomeric material either by injection or compression.

2. Pre-filled syringe (2) according to claim 1, wherein said communication of fluids through a plurality of grooves (9) when the valvular joint (1) is in a half-fitted position (a) allows the lyophilization of the content of the syringe with the valvular joint (1) installed inside the syringe (2).

3. Pre pre-filled syringe (2) according to claim 1, wherein a compact closing cylinder (6) formed from the elastic base (5) of the elastic discoid cap (3) has a circular section, where the quantity of grooves (9) of the plurality of grooves from the distal zone of the compact closing cylinder (6) is from two to six, preferably four.

4. Pre-filled syringe (2) according to claim 1, wherein a compact closing cylinder (6) formed from the elastic base (5) of the elastic discoid cap (3) has a circular section, where the external diameter of the proximal fit area (10) is slightly bigger than the diameter of the central passage tube (7) of the sliding receiving discoid seat (4) providing an adjusted fit.

5. Pre-filled syringe (2) according to claim 1, wherein alternatively the compact closing cylinder (6) that is formed from the elastic base (5) of the valvular cap (3), includes in a proximal fit area (10) a plurality of external annular flanges (11) that are temporary supported by the cylindrical surface that defines the central passage (7) of the sliding receiving discoid seat (4) providing an adjusted fit.

6. Pre-filled syringe (2) according to any of the above claims from 1 to 5, wherein the discoid cooperative elements (3 and 4) are combined with a tip-cap (16) seal that closes the communication tube towards the injection needle (18), which is not pre-installed in its position. This is a temporary closure resource for pre-filled syringes (2) of double internal coaxial chambers (12 and 13), where said discoid cooperative elements (3 and 4) are arranged in a way that they act as a separating partition between both chambers (12 and 13).

7. Pre-filled syringe (2) according to any of the above claims 1 and 3 to 5, wherein the discoid cooperative elements (3 and 4) are arranged as a temporary closure that prevents the main body of the syringe (2) from communicating internally with the communication tube towards the injection needle (18). This could be a temporary closure resource for single pre-filled chamber syringes (2).

8. Pre-filled syringe (2) according to any of the claims 1 to 5, wherein the elastic valvular joint (1) delimits two chambers (12 and 13), each one including a different product (14 and 15), and, at least one of these products is liquid. And upon mixing both products (14 and 15), a solution (22) is obtained to be injected. The elastic base (5) of the elastic discoid cap (3) of the elastic valvular joint (1) is opposed to the head (20) of the plunger (21).

9. Pre-filled syringe (2) according to claim 8, wherein the elastic valvular joint (1) delimits a chamber (23) that contains a solution (22) to be injected, where the elastic base (5) of the elastic discoid cap (3) of the elastic valvular joint (1) faces the head (20) of the plunger (21).

10. Pre-filled syringe (2) according to claim 11, wherein the injection needle (18) is pre-installed in the exit tube (17) of the syringe (2).
